(19) Europäisches Patentamt
European Patent Office
Office européen des brevets

(11) Veröffentlichungsnummer: **0 152 574**
**A1**

(12) **EUROPÄISCHE PATENTANMELDUNG**

(21) Anmeldenummer: **84114982.6**

(22) Anmeldetag: **08.12.84**

(51) Int. Cl.⁴: **C 07 D 277/68**, A 61 K 31/425

(30) Priorität: **17.12.83 DE 3345702**

(43) Veröffentlichungstag der Anmeldung: **28.08.85 Patentblatt 85/35**

(84) Benannte Vertragsstaaten: **AT BE CH DE FR GB IT LI LU NL SE**

(71) Anmelder: **Dr. Karl Thomae GmbH, Postfach 1755, D-7950 Biberach (Riss) (DE)**

(72) Erfinder: **Trummlitz, Günter, Dr. Dipl.-Chem., Buchenweg 27, D-7951 Warthausen (DE)**
Erfinder: **Engel, Wolfhard, Dr.,Dipl.-Chem., Mozartstrasse 13,, D-7950 Biberach 1 (DE)**
Erfinder: **Eberlein, Wolfgang, Dr.,Dipl.-Chem., Obere Au 6, D-7950 Biberach 1 (DE)**
Erfinder: **Schmidt, Günther, Dipl.-Chem., Johann-Sebastian-Bach-Strasse 27, D-7950 Biberach 1 (DE)**
Erfinder: **Prox, Axel, Prof. Dr. Dipl.-Chem., Kapellenweg 13, D-7950 Biberach 1 (DE)**
Erfinder: **Engelhardt, Günther, Prof. Dr., Unterer Bühl 18, D-7950 Biberach 1 (DE)**

(54) **6-Ethoxy-4-methyl-benzothiazol-2(3H)-on, Verfahren zur Herstellung und diese Verbindung enthaltende Arzneimittel.**

(57) Beschrieben wird als neue Substanz das 6-Ethoxy-4-methyl-benzothiazol-2(3H)-on, Verfahren zu seiner Herstellung und diese Verbindung enthaltende Arzneimittel.

Das 6-Ethoxy-4-methyl-benzothiazol-2(3H)-on zeichnet sich durch gute analgetische und antipyretische Wirkungen bei einer guten Leberverträglichkeit aus.

EP 0 152 574 A1

DR. KARL THOMAE GMBH
D-7950 Biberach 1

Case 5/889
Dr. Bu/fra

BEZEICHNUNG GEÄNDERT
siehe Titelseite

Neues Benzothiazol-2(3H)-on, Verfahren zur Herstellung und diese Verbindung enthaltende Arzneimittel

Die Erfindung betrifft als neue Verbindung das 6-Ethoxy-4-methyl-benzothiazol-2(3H)-on der Formel

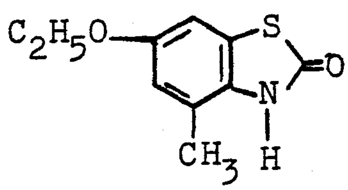

,(I)

Verfahren zu seiner Herstellung und diese Verbindung enthaltende Arzneimittel.

Es sind verschiedene chemisch ähnliche Benzothiazol-2(3H)-one auch als analgetisch und antipyretisch wirkende Substanzen bereits beschrieben worden (vgl. DE-Al-30 17 977.2, Europäische Patentschrift Nr. 0 039 440 und DE-Al-29 27 352.7). Es wurde nun gefunden, daß die neue Verbindung 6-Ethoxy-4-methyl-benzothiazol-2(3H)-on sich gegenüber den vorbekannten Verbindungen bei gleich guten analgetischen und antipyretischen Eigenschaften durch eine wesentlich bessere Leberverträglichkeit auszeichnet.

Das 6-Ethoxy-4-methyl-benzothiazol-2(3H)-on läßt sich nach den folgenden Methoden herstellen:

1.) Ein Benzothiazol der allgemeinen Formel II

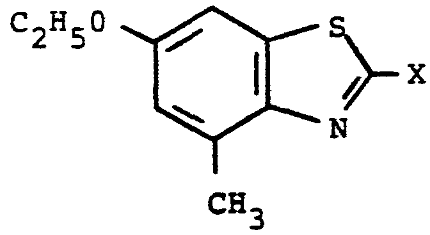

,(II)

in der X ein Chlor- oder Bromatom oder eine bis zu 3 Kohlenstoffatomen enthaltende Alkoxygruppe bedeutet, wird bei Temperaturen zwischen 0 und 120°C, vorzugsweise bei Siedetemperatur des Reaktionsgemisches, durch Behandlung mit wässerigen Mineralsäuren, beispielsweise mit konz. Salzsäure oder Bromwasserstoffsäure, 20 bis 60%iger Schwefelsäure oder halbkonz. Phosphorsäure, zu der gewünschten Verbindung 6-Ethoxy-4-methyl-benzothiazol-2(3H)-on hydrolysiert. Die Reaktion kann in Gegenwart inerter, mit Wasser mischbarer Lösungsmittel, z.B. Methanol, Ethanol, 1-Propanol, 2-Propanol, Tetrahydrofuran, 1,4-Dioxan, 1,2-Ethandiol durchgeführt werden, gelingt jedoch auch bei Abwesenheit zusätzlicher Lösungsmittel.

2.) Das 2(3H)-Benzothiazolthion der Formel III

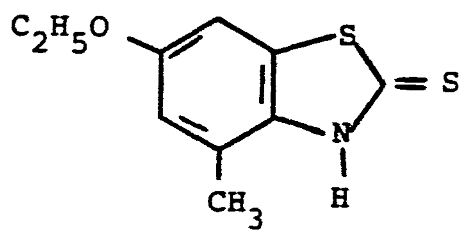

,(III)

wird oxidiert und zu der gewünschten Verbindung 6-Ethoxy-4-methyl-benzothiazol-2(3H)-on hydrolysiert. Als Oxidationsmittel werden Brom, Chlor, Wasserstoffperoxid und Alkalipermanganate bevorzugt. Die Reaktion wird in wässerig alkalischem Medium durchgeführt. Als geeignete Basen seien Natriumhydroxid, Kaliumhydroxid, Calciumhydroxid, Bariumhydroxid genannt. Während bei der Oxidation mit Brom, Chlor und Wasserstoffperoxid, die bei -10 bis +30°C durchgeführt wird, Zwischenprodukte nicht nachgewiesen werden können, führt die Umsetzung mit Alkalipermanganaten, wie Kaliumpermanganat, zunächst zu einer Sulfonsäure der Formel IIIa

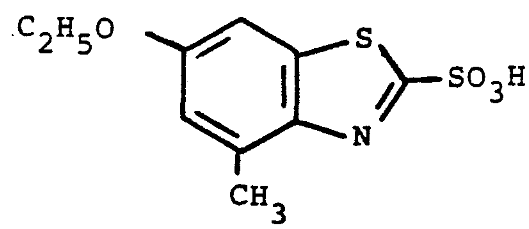

,(IIIa)

die - in der Regel ohne Isolierung - durch Erwärmen mit wäßrigen Mineralsäuren, beispielsweise mit konz. Salz- oder Bromwasserstoffsäure, mit 30- bis 70-%iger Schwefelsäure oder halbkonz. Phosphorsäure, auf Temperaturen zwischen 40 und 100°C in das gewünschte 6-Ethoxy-4-methyl-benzothiazol-2(3H)-on übergeführt wird.

Bei der Oxidation mit Brom oder Chlor sollten zur Vermeidung von Kernhalogenierungen keinesfalls mehr als 3 Mol des Halogens pro Mol 2(3H)-Benzothiazolon der Formel III angewendet werden, während die Reaktion mit Wasserstoffperoxid wenigstens 3 Mol, die mit Permanganaten wenigstens 2 Mol des Oxidationsmittels erfordert, wenn eine quantitative Umsetzung angestrebt wird.

In einer Variante dieses Verfahrens werden Alkalisalze des 2(3H)-Benzothiazolthions der Formel III zunächst mit Alkyljodiden oder Dialkylsulfaten oder Benzylhalogeniden zu 2-substituierten Benzothiazolen der allgemeinen Formel IIIb

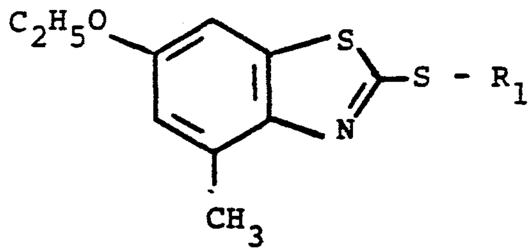

,(IIIb)

in der $R_1$ einen niederen Alkylrest mit 1 bis 3 Kohlenstoffatomen oder einen Benzylrest bedeutet, umgesetzt, anschließend mit Kaliumpermanganat in alkalischer Lösung oder in Eisessig zu Sulfonen der allgemeinen Formel IIIc

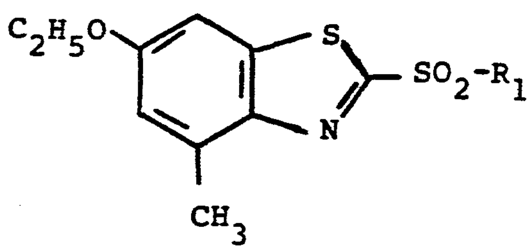

,(IIIc)

oxidiert, und diese schließlich durch Erwärmung auf 40 bis 100°C mit wäßrigen Mineralsäuren in die gewünschte Endverbindung 6-Ethoxy-4-methyl-benzothiazol-2(3H)-on übergeführt. Die Hydrolyse der Sulfone der allgemeinen Formel IIIc wird bevorzugt in Gegenwart von mit Wasser mischbaren Cosolventien, z.B. von Methanol, Ethanol, 1-Propanol, 2-Propanol, 1,4-Dioxan, Tetrahydrofuran durchgeführt, sie gelingt jedoch auch in Abwesenheit zusätzlicher Lösungsmittel.

3.) Das Benzothiazolamin der Formel IV

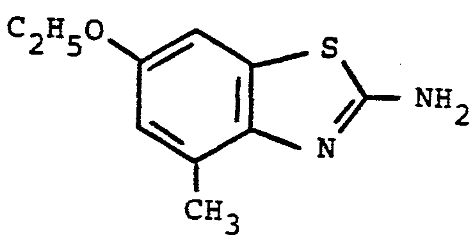

,(IV)

wird mit einer Base, beispielsweise Alkali- oder Erdalkalihydroxid, in einem alkalibeständigen Lösungs- oder Verdünnungsmittel in Abwesenheit von Wasser oder unter weitgehendem Ausschluß von Wasser bei Temperaturen bis zum Siedepunkt des Reaktionsgemisches umgesetzt und das entstandene Alkali- oder Erdalkalisalz des o-Mercaptophenylharnstoffes der Formel IVa

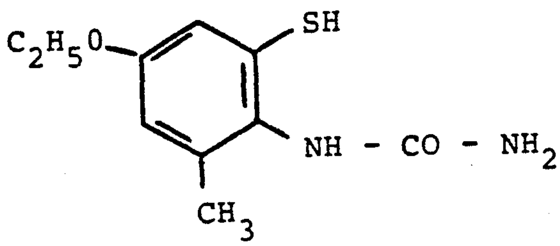

,(IVa)

vorteilhafterweise ohne Zwischenisolierung, durch Behandlung mit einer Säure zum gewünschten 6-Ethoxy-4-methylbenzothiazol-2(3H)-on cyclisiert.

Die Ringöffnung zu der Verbindung der Formel IVa gelingt beispielsweise mit wenigstens äquimolaren Mengen an Lithiumhydroxid, Calciumhydroxid, Bariumhydroxid, bevorzugt jedoch Kaliumhydroxid oder Natriumhydroxid, in Lösungsmitteln, die unter alkalischen Bedingungen stabil sind, wie ein- oder mehrwertige Alkohole, z.B. Isobutanol, 1,2-Ethandiol, 1,3-Propandiol, Glycerin, oder Monoalkyläther der genannten Diole oder Triole, oder in Gemischen dieser Lösungsmittel durch Erhitzen auf Temperaturen von 80-200°C, bevorzugt 120-160°C. Die Abwesenheit von Wasser bei dieser Reaktion garantiert hohe Ausbeuten. Die Umsetzung kann zwar auch in Gegenwart der geringen Wassermengen, die üblicherweise in den genannten Lösungsmitteln enthalten sind, durchgeführt werden, eine Verminderung der Ausbeute und Reinheit des Endproduktes ist jedoch dann in Kauf zu nehmen.

Der o-Mercapto-phenylharnstoff der Formel IVa kann durch Zugabe der äquivalenten Menge Säure, bezogen auf die eingesetzte Menge Alkali- oder Erdalkalihydroxid, freigesetzt und durch Filtration abgetrennt werden, es ist jedoch von Vorteil das Salz oder die freie Verbindung im Lösungs- oder Verdünnungsmittel direkt weiterzuverarbeiten.

Die Cyclisierung des o-Mercapto-phenylharnstoffs der Formel IVa gelingt mit katalytischen oder auch überschüssigen Mengen an Säuren. Die Cyclisierung kann in einem der obengenannten Lösungs- oder Verdünnungsmittel erfolgen, sie gelingt jedoch auch ohne Verwendung eines solchen. Als Säuren werden wässerige Mineralsäuren, wie Salzsäure, Schwefelsäure, Phosphorsäure, insbesondere 10 bis 30%ige Salzsäure bevorzugt. Die Cyclisierung tritt bei Temperaturen zwischen 40-100°C, bevorzugt 70-100°C ein.

4.) Das 2-Aminothiophenol der Formel V

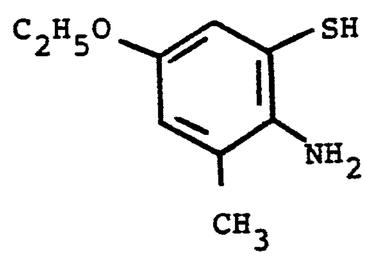

,(V)

wird mit einem Kohlensäurederivat der allgemeinen Formel VI

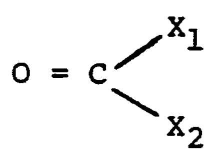

(VI)

in der $X_1$ und $X_2$ gleich oder verschieden sind und Halogen, insbesondere Chlor; die 1-Imidazolyl-; 1,2,4-Triazol-4-yl-; oder die 1,2,4-Triazol-1-ylgruppe; eine Alkoxygruppe mit 1 bis 10 Kohlenstoffatomen, wie die Methoxy-, Ethoxy-, Propoxy-, Isopropoxy-, Butoxy-, Pentyloxy-, die Phenoxy- oder p-Tolyloxygruppe; die Benzyloxy-, Phenethyloxygruppe oder zusammen ein Schwefelatom bedeuten, in einem Lösungsmittel bei Temperaturen zwischen 0 und 150°C, vorzugsweise zwischen 20°C und der Siedetemperatur des Reaktionsgemisches, und gegebenenfalls in Gegenwart zusätzlicher anorganischer oder organischer Basen, z.B. von Alkali- oder Erdalkalihydroxiden, -carbonaten, -hydrogencarbonaten oder -alkoholaten, von tert. Aminen wie Pyridin, Triethylamin, N,N-Dimethylanilin, N,N-Diethylanilin oder von quartären Ammoniumhydroxiden, z.B. von Benzyltrimethylammoniumhydroxid, cyclisiert.

Als Lösungsmittel eignen sich Wasser, Alkohole wie Methanol, Ethanol, 1-Propanol, 2-Propanol; bevorzugt werden jedoch wasserfreie aprotische Lösungsmittel wie Benzol, Toluol oder andere Kohlenwasserstoffe, Tetrahydrofuran, 1,4-Dioxan oder andere cyclische Ether, Dimethylformamid, Dimethylsulfoxid. Auch Gemische der genannten Lösungsmittel können verwendet werden.

In einer Variante dieses Verfahrens verwendet man als Kohlensäurederivate Chlorkohlensäureester der allgemeinen Formel VIa

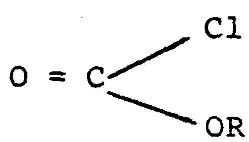

(VIa)

in der R einen Alkylrest mit 1 bis 10 Kohlenstoffatomen, beispielsweise die Methyl-, Ethyl-, 1-Propyl-, 2-Propyl-, 1-Butyl-, 2-Methyl-1-propyl-, Dimethylethyl-, 1-Pentyl- oder 1-Hexylgruppe, oder einen Arylrest, wie die Phenyl- oder 4-Methylphenylgruppe oder einen Aralkylrest mit 1 bis 3 Kohlenstoffatomen in der Alkylengruppe, wie die Phenylmethyl- oder 2-Phenylethylgruppe bedeutet; dabei entstehen zunächst Zwischenverbindungen der allgemeinen Formel VII

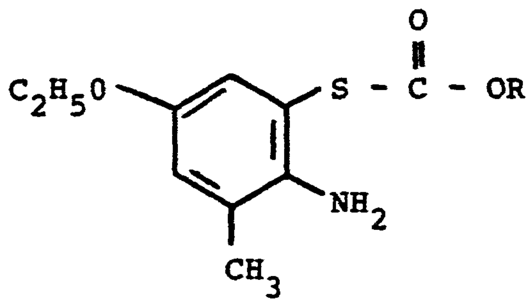

,(VII)

in der R wie oben definiert ist.

Diese Zwischenverbindungen werden anschließend in Gegenwart saurer Katalysatoren zu der gesuchten Verbindung 6-Ethoxy-4-methyl-benzothiazol-2(3H)-on cyclisiert. Als saure Katalysatoren werden starke oder mittelstarke Mineralsäuren bevorzugt, z.B. Salzsäure, Bromwasserstoffsäure, Schwefelsäure, Phosphorsäure, Die Cyclisierungsreaktion wird in der Regel in inerten organischen Lösungsmitteln durchgeführt, beispielsweise in Methanol, Ethanol, Toluol, Essigsäure, Propionsäure. Die Reaktionstemperaturen liegen zwischen 20 und 150°C, vorzugsweise beim Siedepunkt des verwendeten Lösungsmittels.

Die Ausgangsstoffe der allgemeinen Formel II, in der X ein Chlor- oder Bromatom bedeutet, lassen sich in Anlehnung an die von G. Mazzone und G. Pappalardo in Il Pharmaco, Ed. Sc. 32, 348-354 (1977) beschriebene Methode aus dem entsprechend substituierten 2-Benzothiazolamin durch Umsetzung mit warmer konzentrierter Halogenwasserstoffsäure und Natriumnitrit bereiten.

Die Ausgangsstoffe der allgemeinen Formel II, in der X eine Alkoxygruppe ist, sind aus entsprechend substituierten N-Aryl-thiourethanen durch Umsetzung mit heißer wäßrig-alkoholischer Lösung von Kaliumhexacyanoferrat(III) und Natriumhydroxid (analog R.F. Hunter und E.R. Parken, J. chem. Soc. (London) 1935, 1755-1761) oder aus Verbindungen der allgemeinen Formel II, in der X ein Chlor- oder Bromatom bedeutet, durch Reaktion mit Alkali- oder Erdalkalialkanolaten, vorzugsweise in den entsprechenden siedenden Alkoholen, zugänglich.

Das als Ausgangsstoff benötigte 6-Ethoxy-4-methyl-benzothiazol-2(3H)-thion der Formel III erhält man in Analogie zu Literaturangaben (L.B. Sebrell und C.E. Boord, J. Amer. Chem. Soc. 49, 1748-1758 (1927)) beispielsweise aus geeigneten Anilinen, Schwefelkohlenstoff und Schwefel unter Druck und bei erhöhter Temperatur oder aus geeigneten o-Halogennitrobenzolen, Natriumsulfid, Schwefelwasserstoff und Schwefelkohlenstoff.

Das 2-Amino-6-ethoxy-4-methyl-benzothiazol der Forml IV kann in Analogie zu literaturbekannten Methoden synthetisiert werden (z.B. H.P. Kaufmann et al., Arch. Pharm. 273, 31 (1935); H.P. Kaufmann, Ber. dtsch. chem. Ges. 62 B, 390 (1929); R.L. Mital und S.K. Jain, J. Chem. Soc. (C) 1969, 2148-2149).

Das als Ausgangsstoff dienende 2-Aminothiophenol der Formel V erhält man aus dem 6-Ethoxy-4-methyl-2-benzothiazolamin durch Hydrolyse mit siedender wäßriger Kalilauge (analog R.L. Mital und S.K. Jain, J. Chem. Soc. (C) 1969, 2148, bzw. S.K. Jain und R.L. Mital, Z. Naturforsch. 32 B , 821 (1977) bzw. H. Hauser, Helv. Chim. Acta 11, 208 (1928) bzw. R. Schuloff et al., Ber. dtsch. Chem. Ges. 61, 2541 (1928)).

Die Ausgangsverbindungen der allgemeinen Formeln VI und VIa sind literaturbekannt.

Das 6-Ethoxy-4-methyl-benzothiazol-2(3H)-on ist pharmakologisch wirksam und besitzt eine sehr gute analgetische und antipyretische Wirkung ohne die ausgeprägten Nebenwirkungen, insbesondere auf die Leber, der chemisch nächstverwandten Verbindungen zu zeigen.

Nachstehend werden die Resultate der vergleichenden Untersuchungen folgender Substanzen mitgeteilt:

6-Ethoxy-4-methyl-benzothiazol-2(3H)-on = A,

6-Methoxy-4-methyl-benzothiazol-2(3H)-on
(siehe DE-A1-30 17 977.2) = B,

4,6-Dimethyl-benzothiazol-2(3H)-on
(siehe DE-A1-30 17 977.2) = C
und
Paracetamol (p-Hydroxy-acetanilid) = D.

Die genannten Verbindungen wurden vergleichend auf ihre analgetische und ihre antipyretische Wirkung an der Ratte, ihre akute Toxizität an der Ratte und ihre Wirkung auf den Glutathiongehalt der Mäuseleber untersucht.

<u>Methodik</u>

1.) Die Prüfung der <u>Wirkung gegen</u> den <u>Entzündungsschmerz</u> der <u>Ratte</u> erfolgte mit der Methode nach RANDALL u. SELITTO (Arch. int. Pharmacodyn. <u>111</u>, 409 (1957)). Die Prüfsubstanzen wurden männlichen Ratten mit einem Gewicht zwischen 100 und 130 g als Verreibung in 1%iger Methylzellulose (1,0 ml/100 g Tier) 90 Minuten nach der subplantaren Hefegabe per Schlundsonde verabfolgt. Aus der 90 Minuten nach Gabe der verschiedenen Dosen der Prüfsubstanz gemessenen Schmerzschwelle wurde nach linearer Regressionsanalyse eine $ED_{50}$ als jene Dosis ermittelt, die eine Anhebung der Schmerzschwelle um 50 % bewirkt.

2. Die Prüfung der antipyretischen Wirkung erfolgte gegen das durch subcutane Gabe von 100 mg Hefe/100 g Tier ausgelöste Fieber an 120-150 g schweren männlichen Ratten. Die Prüfsubstanzen wurden 4 Stunden nach Hefegabe als Verreibung in 1%iger Methylzellulose (1,0 ml/100 g) per Schlundsonde verabfolgt. Aus der 2 Stunden danach gefundenen Veränderung der Rektaltemperatur gegenüber der der Kontrolltiere wurde nach linearer Regressionsanalyse eine $ED_{-1,0°C}$ als jene Dosis berechnet, die das Hefefieber um 1°C senkte.

3.) Die Bestimmung der akuten Toxizität erfolgte an Ratten beider Geschlechter mit einem Gewicht zwischen 120 und 150 g. Die Prüfsubstanzen wurden als Verreibung in 1%iger Methylzellulose (2,0 ml/100 g Tier) per Schlundsonde appliziert. Die Berechnung der $LD_{50}$ erfolgte nach LITCHFIELD u. WILCOXON (J. Pharmacol. exp. Ther. 96, 99 (1949)) aus dem Prozentsatz der Tiere, die nach den verschiedenen Dosen innerhalb von 14 Tagen verstarben.

4.) Der Einfluß auf den Glutathion-Gehalt der Leber wurde an männlichen Mäusen mit einem Gewicht zwischen 25 und 30 g untersucht. 2 Stunden nach oraler Gabe der Prüfsubstanz (als Verreibung in 1%iger Methylzellulose, 0,2 ml/10 g Tier) wurde die Leber entnommen. Die Bestimmung des Glutathion erfolgte mit der Methode von ELLMAN (Arch. Biochem. Biophys. 82, 70 (1959)).

Ergebnisse

Die bei diesen Prüfungen erhobenen Befunde sind in den Tabellen 1 bis 4 zusammengestellt.

Die Substanz A zeigt eine dem Paracetamol überlegene analgetische und antipyretische Wirkung bei geringerer Toxizität.

Die starke analgetischen und antipyretischen Eigenschaften teilt die Substanz A mit den Substanzen B und C. Von diesen beiden Verbindungen B und C unterscheidet sich A durch eine bessere Leberverträglichkeit.

Für die prognostische Beurteilung einer Hepatotoxizität ist die Erschöpfung der Glutathionvorräte in der Leber ein geeigneter Indikator. Am Beispiel des Paracetamol läßt sich zeigen, daß eine Verarmung der Glutathionvorräte in der Leber der Maus um mehr als 50 % gleichbedeutend mit dem Beginn des Leberzelluntergangеs ist (MITCHELL et al., J. Pharmacol. exp. Therap. 187, 211 (1973)). Während die Substanzen B und C in einer oralen Einzeldosis von 1000 mg/kg das Leberzellglutathion um mehr als 50 % vermindern, bleibt der Einfluß von Substanz A auf das Leberzellglutathion unter den gleichen Versuchsbedingungen deutlich schwächer und unterhalb des kritischen Bereiches.

Tabelle 1:

Wirkung gegen den Entzündungsschmerz der Ratte in der Versuchsanordnung nach RANDALL u. SELITTO 90 Minuten nach oraler Gabe

| Substanz | $n_1$* | $n_2$* | $ED_{50}$ mg/kg | |
|---|---|---|---|---|
| A | 3 | 10 | 24,1 | (20,3-29,9) |
| B | 3 | 10 | 23.0 | (21,8-24,2) |
| C | 3 | 10 | 49,7 | (46,6-53,1) |
| D | 3 | 20 | 215 | (202 - 230) |

*$n_1$ = Anzahl der geprüften Dosen; $n_2$ = Anzahl der Tiere/Dosis.

Tabelle 2:

Wirkung gegen das Hefefieber der Ratte nach oraler Gabe:

| Substanz | $n_1$ * | $n_2$* | $ED_{-1,0}$ °C | |
|---|---|---|---|---|
| A | 3 | 11 | 31,2 | (25,1-36,5) |
| B | 3 | 12 | 29,6 | (25,0-34,0) |
| C | 4 | 10 | 18,1 | (11,3-23,7) |
| D | 3 | 10 | 123 | (84,0-155) |

* Legende siehe Tabelle 1

Tabelle 3

Akute Toxizität an der Ratte nach oraler Gabe (bei einer Nachbeobachtungszeit von 14 Tagen).

| Substanz | $n_1$ * | $n_2$* | $LD_{50}$ mg/kg |
|---|---|---|---|
| A | 3 | 10 | > 4 000 ** |
| B | 3 | 10 - 20 | 7580 (6534-8792) |
| C | 4 | 10 | 2920 (2517-3387) |
| D | 3 | 10 | 3959 (3299-4751) |

* Legende siehe Tabelle 1
** nach dieser Dosis 0/10 Tieren verstorben!

Tabelle 4:

Wirkung auf den Glutathiongehalt der Mäuseleber 2 Stunden nach oraler Gabe

| Substanz | Dosis mg/kg | n | red. GSH mMol/g $\bar{x}$ | SE | Δ gegen Kontrollen [ % ] |
|---|---|---|---|---|---|
| Kontrollen | - | 44 | 7,84 | 0,19 | - |
| D | 160 | 8 | 5,28 | 0,37 | - 32,7 |
| | 200 | 8 | 3,82 | 0,36 | - 51,3 |
| | 250 | 8 | 2,69 | 0,42 | - 65,7 |
| | 315 | 8 | 1,38 | 0,29 | - 82,4 |
| A | 1000 | 6 | 5,02 | 0,43 | - 36,0 |
| B | 1000 | 6 | 3,42 | 0,19 | - 56,4 |
| C | 1000 | 6 | 2,90 | 0.20 | - 63,0 |

$\bar{x}$ = Mittelwert, SE = Standard Error, red GSG = reduziertes Glutathion

Die nachfolgenden Beispiele sollen die Erfindung näher erläutern:

Beispiel 1

6-Ethoxy-4-methyl-benzothiazol-2(3H)-on

20,4 g (0,09 Mol) 6-Ethoxy-2-methoxy-4-methyl-benzothiazol wurden in 200 ml Ethanol zum Sieden erhitzt und tropfenweise mit 80,5 ml konzentrierter Salzsäure versetzt und 1 Stunde am Rückfluß erhitzt. Danach wurde nach dem Abkühlen im Eisbad der entstandene Niederschlag abfiltriert und mit Wasser gewaschen. Nach Umkristallisation aus Ethanol wurden 15,8 g (84 % der Theorie) 6-Ethoxy-4-methyl-benzothiazol-2(3H)-on erhalten;

Schmelzpunkt 193-194°C

$C_{10}H_{11}NO_2S$ (209,27)

| | | | | | | | | |
|---|---|---|---|---|---|---|---|---|
| Ber.: | C | 57,39 | H | 5,30 | N | 6,69 | S | 15,32 |
| Gef.: | | 57,16 | | 5,18 | | 6,47 | | 15,25 |

Die Herstellung der Ausgangsverbindung erfolgte auf den folgenden Wegen:

A) Aus (4-Ethoxy-2-methylphenyl)-thiocarbaminsäure-O-methylester

(4-Ethoxy-2-methylphenyl)-thiocarbaminsäure-O-methylester wurde mit Kaliumhexacyanoferrat-(III) in methanolischer Natronlauge (in Analogie zu R.F. Hunter und E.R. Parken, J. Chem. Soc. (London) 1935, 1755) umgesetzt.

B) Aus 2-Chlor-6-ethoxy-4-methyl-benzothiazol

2-Chlor-6-ethoxy-4-methyl-benzothiazol wurde mit Natriummethylat durch Erhitzen am Rückfluß in 92%iger Ausbeute zu 6-Ethoxy-2-methoxy-4-methyl-benzothiazol umgesetzt. Das 2-Chlor-6-ethoxy-4-methyl-benzothiazol wurde durch Diazotierung und anschließende Umsetzung mit Kupfer-(I)-chlorid aus 2-Amino-6-ethoxy-4-methyl-benzothiazol hergestellt.

Beispiel 2

6-Ethoxy-4-methyl-benzothiazol-2(3H)-on

Hergestellt analog Beispiel 1 aus 2,6-Di-ethoxy-4-methyl-benzothiazol in 79 % Ausbeute;
Schmelzpunkt: 193-194°C.
Nach Mischschmelzpunkt, Elementaranalyse, Dünnschichtchromatographie und IR- und NMR-Spektrum mit dem Präparat nach Beispiel 1 identisch.

Beispiel 3

6-Ethoxy-4-methyl-benzothiazol-2(3H)-on

45,5 g (0,2 Mol) 2-Chlor-6-ethoxy-4-methyl-benzothiazol wurden in einer Mischung aus 500 ml Ethanol und 500 ml konzentrierter wäßriger Salzsäure 5 Stunden am Rückfluß erhitzt. Nach dem Abkühlen wurde der Niederschlag abfiltriert, in verdünnter Natronlauge aufgenommen und durch Ansäuern ausgefällt. Nach Umkristallisation aus Ethanol wurden 29,2 g (70 % der Theorie) 6-Ethoxy-4-methyl-benzothiazol-2(3H)-on erhalten;
Schmelzpunkt: 193-194°C.

Nach Mischschmelzpunkt, Elementaranalyse, Dünnschichtchromatographie und IR- und NMR-Spektrum mit dem Präparat nach Beispiel 1 identisch.

Beispiel 4

6-Ethoxy-4-methyl-benzothiazol-2(3H)-on

In eine Lösung von 30,0 g (0,8 Mol) Natriumhydroxid in 400 ml Wasser wurden 123 g (0,5 Mol) 6-Ethoxy-4-methyl-benzothiazol-2(3H)-thion eingetragen. Innerhalb von 2 bis 3 Stunden wurde dann bei 25 bis 30°C eine Kaliumpermanganatlösung, die durch Auflösen von 205,5 g (1,3 Mol) Kaliumpermanganat in 2 l Wasser bei 40°C erhalten wurde, unter Rühren zugetropft. Nach beendeter Zugabe wurde auf 80°C erwärmt und danach vom entstandenen Braunstein abgesaugt. Der Filterrückstand wurde in 1 l Wasser aufgenommen, auf 80°C erwärmt und heiß filtriert. Die vereinigten wäßrigen Filtrate wurden auf 70°C erwärmt, mit 200 ml konzentrierter wäßriger Salzsäure versetzt und 6 Stunden unter Rückfluß gekocht. Nach dem Abkühlen des Reaktionsgemisches wurde vom Niederschlag abfiltriert. Das Material wurde aus Ethanol umkristallisiert und ergab 82,0 g (78 % der Theorie) 6-Ethoxy-4-methyl-benzothiazol-2(3H)-on.

Schmelzpunkt: 193-194°C.

$C_{10}H_{11}NO_2S$ (209,27)

| | | | | | | | | |
|---|---|---|---|---|---|---|---|---|
| Ber.: | C | 57,39 | H | 5,30 | N | 6,69 | S | 15,32 |
| Gef.: | | 57,26 | | 5,10 | | 6,47 | | 15,20 |

Beispiel 5

6-Ethoxy-4-methyl-benzothiazol-2(3H)-on

225 g (1 Mol) 6-Ethoxy-4-methyl-benzothiazol-2(3H)-thion wurden in eine Lösung von 120 g (3 Mol) Ätznatron in 1,5 l

Wasser eingetragen und bei einer Temperatur von 20 bis 30°C unter Rühren mit 340 g 30-%iger Wasserstoffperoxid-Lösung versetzt. Es wurde weitere 6 Stunden bei 40°C gerührt und nach dem Erkalten mit Salzsäure auf pH 6,5 gestellt. Der Niederschlag wurde abfiltriert und aus Ethanol umkristallisiert. Man erhielt 195 g (93 % der Theorie) 6-Ethoxy-4-methyl-benzothiazol-2(3H)-on;
Schmelzpunkt: 193-194°C.
Nach Mischschmelzpunkt, Elementaranalyse, Dünnschichtchromatogramm und IR-, UV-, NMR- und MS Spektrum mit dem Präparat nach Beispiel 4 identisch.

## Beispiel 6

### 6-Ethoxy-4-methyl-benzothiazol-2(3H)-on

In eine Lösung von 320 g (8 Mol) Natriumhydroxid und 225 g (1 Mol) 6-Ethoxy-4-methyl-benzothiazol-2(3H)-thion in 5 l Wasser wurden bei einer Temperatur von -10 bis +10°C 213 g (3 Mol) Chlor eingeleitet. Der entstandene Niederschlag wurde abfiltriert, mit Wasser gewaschen, aus Ethanol umkristallisiert und ergab 183 g (87 % der Theorie) 6-Ethoxy-4-methyl-benzothiazol-2(3H)-on;
Schmelzpunkt: 193-194°C.
Das Produkt war nach Mischschmelzpunkt und Dünnschichtchromatogramm mit dem nach Beispiel 4 erhaltenen Material identisch.

Beispiel 7

6-Ethoxy-4-methyl-benzothiazol-2(3H)-on

11,21 g (0,05 Mol) 6-Ethoxy-4-methyl-benzothiazol-2(3H)-thion werden mit 100 ml einer 1 N Natriumhydroxid-Lösung versetzt und nach Zugabe einer Lösung von 4,0 ml (9,12 g, entsprechend 0,064 Mol) Methyljodid in 10 ml Ethanol 15 Minuten bei Zimmertemperatur kräftig gerührt. Nach 1-stündigem Stehenlassen wurde das Reaktionsprodukt abfiltriert, mit Wasser gewaschen und bei 70°C getrocknet. Dieses Zwischenprodukt (6-Ethoxy-4-methyl-2-methylthiobenzothiazol) wurde in 40 ml Eisessig gelöst. Unter Rühren wurde eine Lösung von 8,25 g (0,052 Mol) Kaliumpermanganat in 125 ml Wasser eingetropft. Nach 1 Stunde Rühren bei Raumtemperatur wurde unter äußerer Eiskühlung Schwefeldioxid durch die Mischung geleitet, bis die braune Färbung verschwunden war. Das entstandene Zwischenprodukt (6-Ethoxy-4-methyl-2-methylsulfonylbenzothiazol) wurde abfiltiert und direkt in 40 ml konzentrierter wäßriger Salzsäure, die mit 10 ml Ethanol versetzt wurde, 4 Stunden am Rückfluß erhitzt. Danach wurde mit 200 ml Wasser versetzt und durch Zugabe von wäßriger Natriumhydroxid-Lösung alkalisch gestellt. Es wurde filtriert, das Filtrat mit Essigsäure angesäuert und der entstandene Niederschlag abfiltriert. Nach Waschen mit Wasser und Trocknen wurde aus Ethanol umkristallisiert und 7,1 g (68 % der Theorie) 6-Ethoxy-4-methyl-benzothiazol-2(3H)-on erhalten.
Schmelzpunkt: 192-194°C
Das Produkt war dünnschichtchromatographisch und analytisch mit dem nach Beispiel 4 erhaltenen Produkt identisch.

Beispiel 8

6-Ethoxy-4-methyl-benzothiazol-2(3H)-on

Eine Mischung aus 40 g (0,19 Mol) 2-Amino-6-ethoxy-4-methyl-benzothiazol und 36 g (0,9 Mol) Natriumhydroxid wurde in 100 ml Glykol 6 Stunden bei 130°C gerührt. Die auf 80°C

abgekühlte Lösung wurde in 1 l Eiswasser eingerührt und filtriert. Das Filtrat wurde auf pH 5 gestellt und das ausgefallene Produkt wurde in 200 ml 10%-iger Salzsäure verteilt. Es wurde 1 Stunde bei 90°C gerührt und nach dem Abkühlen wurde das Produkt abfiltriert, getrocknet und aus Ethanol umkristallisiert. Es wurden 21,5 g (54 % der Theorie) 6-Ethoxy-4-methyl-benzothiazol-2(3H)-on erhalten.
Schmelzpunkt: 193-194°C.

$C_{10}H_{11}NO_2S$ (209,27)

| | | | | | | | | |
|---|---|---|---|---|---|---|---|---|
| Gef.: | C | 57,39 | H | 5,30 | N | 6,69 | S | 15,32 |
| Gef.: | | 57,18 | | 5,23 | | 6,41 | | 15,30 |

Beispiel 9

6-Ethoxy-4-methyl-benzothiazol-2(3H)-on

In eine Lösung von 18,3 g (0,1 Mol) 2-Amino-5-ethoxy-3-methyl-thiophenol in 100 ml wasserfreiem Tetrahydrofuran wurden unter Rühren bei einer Temperatur von 50°C portionsweise 17,6 g (0,11 Mol) N,N'-Carbonyl-diimidazol gegeben. Anschließend wurde 1 Stunde unter Rückfluß erhitzt, das Lösungsmittel weitgehend abdestilliert und filtriert. Die Lösung wurde in Wasser eingerührt und das Kristallisat abfiltriert und aus Ethanol zweimal umkristallisiert. Es wurden 8,3 g (40 % der Theorie) 6-Ethoxy-4-methyl-benzothiazol-2(3H)-on erhalten;
Schmelzpunkt: 193-194°C.

$C_{10}H_{11}NO_2S$ (209,27)

| | | | | | | | | |
|---|---|---|---|---|---|---|---|---|
| Ber.: | C | 57,39 | H | 5,30 | N | 6,69 | S | 15,32 |
| Gef.: | | 59,42 | | 5,31 | | 6,51 | | 15,20 |

Die Herstellung des Ausgangsmaterials erfolgte auf dem folgenden Wege:

2-Amino-5-ethoxy-3-methyl-thiophenol

2-Amino-6-ethoxy-4-methyl-benzothiazol wurde in siedender wäßriger Kalilauge 8 Stunden erhitzt (analog R.L. Mital und S.K. Jain, J. Chem. Soc. (c) 1969, 2148) und lieferte nach der Aufarbeitung das 2-Amino-5-ethoxy-3-methyl-thiophenol.

Beispiel 10

6-Ethoxy-4-methyl-benzothiazol-2(3H)-on

In eine Lösung von 9,15 g (0,05 Mol) 2-Amino-5-ethoxy-3-methyl-thiophenol und 5,0 g (0,05 Mol) Triethylamin in 100 ml wasserfreiem Tetrahydrofuran wurden 3,3 g (0,055 Mol) Carbonylsulfid unter Kühlung eingeleitet. Anschließend wurde das Reaktionsgemisch 12 Stunden bei Raumtemperatur gerührt und 8 Stunden unter Rückfluß erhitzt. Nach dem Abkühlen wurde der Niederschlag abfiltriert, aus Ethanol umkristallisiert und lieferte 7,1 g (68 % der Theorie) 6-Ethoxy-4-methyl-benzothiazol-2(3H)-on;
Schmelzpunkt: 192-194°C.
Nach Mischschmelzpunkt und Dünnschichtchromatographie war das erhaltene Produkt mit dem nach Beispiel 9 erhaltenen Material identisch.

Beispiel 11

6-Ethoxy-4-methyl-benzothiazol-2(3H)-on

Zu einer gekühlten Lösung von 20 g (0,5 Mol) Natriumhydroxid in 1 l Wasser wurden 91,5 g (0,5 Mol) 2-Amino-5-ethoxy-3-methyl-thiophenol und 10 g einer 40-%igen methanolischen Lösung von Benzyltrimethylammoniumhydroxid gegeben. Bei einer Reaktionstemperatur von 5°C wurden innerhalb von 2 Stunden

87 g (0,8 Mol) Chlorkohlensäureethylester eingetropft. Das Reaktionsgemisch wurde anschließend 1 Stunde bei Raumtemperatur gerührt. Der entstandene Niederschlag wurde abfiltriert, gewaschen und getrocknet. Das rohe 4-Ethoxy-2-ethoxycarbonylthio-6-methyl-anilin wurde in einer Mischung aus 200 ml Methanol und 30 ml konzentrierter Schwefelsäure 40 Minuten unter Rückfluß gekocht. Nach dem Erkalten wurde in Eiswasser eingerührt und natronalkalisch gestellt. Nach Filtrieren wurde das Filtrat mit Ether ausgezogen und die wäßrige Phase wurde mit 2 N-Salzsäure auf pH 7 gebracht. Der entstandene Niederschlag wurde abfiltriert, getrocknet und aus Ethanol umkristallisiert. Man erhielt 71,0 g (68 % der Theorie) 6-Ethoxy-4-methyl-benzothiazol-2(3H)-on; Schmelzpunkt: 193-194°C.
Das Produkt war dünnschichtchromatographisch und analytisch mit dem unter Beispiel 9 erhaltenen identisch.

Das 6-Ethoxy-4-methyl-benzothiazol-2(3H)-on läßt sich in die üblichen pharmazeutischen Zubereitungsformen, wie Tabletten, Dragées, Suppositorien, Kapseln oder Säfte einarbeiten. Die Einzeldosierung beträgt hierbei für Erwachsene 20 bis 600 mg, vorzugsweise 50 bis 300 mg, dies entspricht einer Tagesdosierung von 60 bis 1800 mg, vorzugsweise von 150 bis 900 mg.

Die nachfolgenden Beispiele sollen die Herstellung einiger pharmazeutischer Zubereitungsformen verdeutlichen.

## Beispiel I

### Tabletten mit 50 mg 6-Ethoxy-4-methyl-benzothiazol-2(3H)-on

Zusammensetzung:
1 Tablette enthält:

| | |
|---|---|
| Wirkstoff | 50,0 mg |
| Milchzucker | 128,0 mg |
| Kartoffelstärke | 40,0 mg |
| Magnesiumstearat | 2,0 mg |
| | 220,0 mg |

### Herstellungsverfahren:

Aus Kartoffelstärke wird durch Erwärmen ein 10%iger Schleim hergestellt. Die Wirksubstanz, Milchzucker und die restliche Kartoffelstärke werden gemischt und mit obigem Schleim durch ein Sieb der Maschenweite 1,5 mm granuliert. Das Granulat wird bei 45°C getrocknet, nochmals durch obiges Sieb gerieben, mit Magnesiumstearat vermischt und zu Tabletten verpreßt.

| | |
|---|---|
| Tablettengewicht: | 220 mg |
| Stempel: | 9 mm |

Beispiel II

Dragees mit 50 mg 6-Ethoxy-4-methyl-benzothiazol-2(3H)-on

Die nach Beispiel I hergestellten Tabletten werden nach bekannten Verfahren mit einer Hülle überzogen, die im wesentlichen aus Zucker und Talkum besteht. Die fertigen Dragees werden mit Hilfe von Bienenwachs poliert.

Drageegewicht: 300 mg

Beispiel III

Kapseln mit 50 mg 6-Ethoxy-4-methyl-benzothiazol-2(3H)-on

Zusammensetzung:
1 Kapsel enthält:

| | |
|---|---|
| Wirkstoff | 50,0 mg |

Herstellungsverfahren:

Der Wirkstoff wird mikronisiert und in Kapseln abgefüllt, letztere werden anschließend verschlossen.

Beispiel IV

Suppositorien mit 50 mg 6-Ethoxy-4-methyl-benzothiazol-2(3H)-on

Zusammensetzung:
1 Zäpfchen enthält:

| | |
|---|---|
| Wirkstoff | 50,0 mg |
| Zäpfchenmassen (z.B. Witepsol W 45 $^{R}$) | 1 650,0 mg |
| | 1 700,0 mg |

Herstellungsverfahren:

Die feinpulverisierte Wirksubstanz wird in der geschmolzenen und auf 40°C abgekühlten Zäpfchenmasse suspendiert. Man gießt die Masse bei 37°C in leicht vorgekühlte Zäpfchenformen aus.

Zäpfchengewicht: 1,7 g

Beispiel V

Tabletten zu 200 mg 6-Ethoxy-4-methyl-benzothiazol-2(3H)-on

1 Tablette enthält:

| | |
|---|---|
| Wirksubstanz | 200,0 mg |
| Milchzucker | 120,0 mg |
| Maisstärke | 70,0 mg |
| Polyvinylpyrrolidon | 8,0 mg |
| Magnesiumstearat | 2,0 mg |
| | 400,0 mg |

Herstellung:

Die Wirksubstanz, der Milchzucker und die Maisstärke werden mit einer wäßrigen Lösung von Polyvinylpyrrolidon gleichmäßig befeuchtet, durch ein Sieb mit 2 mm-Maschenweite gesiebt und im Umlufttrockenschrank bei 50°C getrocknet. Nach erneuter Siebung durch ein Sieb von 1,5 mm-Maschenweite wird Magnesiumstearat zugemischt und die Mischung zu Tabletten verpreßt.

Tablettengewicht: 400 mg

Durchmesser: 11 mm, rund, biplan, beidseitige Facette und einseitige Teilkerbe.

Beispiel VI

Suppositorien zu 200 mg 6-Ethoxy-4-methyl-benzothiazol-2(3H)-on

1 Zäpfchen enthält:

| | |
|---|---|
| Wirksubstanz | 0,20 mg |
| Hartfett (z.B. Witepsol H 19 oder Witepsol W 45) | 1,50 g |
| | 1,70 g |

Herstellung:

Das Hartfett wird geschmolzen. Bei 38°C wird die gemahlene Wirksubstanz in der Schmelze homogen dispergiert. Es wird auf 35°C abgekühlt und in schwach vorgekühlte Suppositorienformen ausgegossen.

Zäpfchengewicht: 1,7 g.

Beispiel VII

Suspension mit 200 mg 6-Ethoxy-4-methyl-benzothiazol-2(3H)-on

100 ml Suspension enthalten:

| | | |
|---|---|---|
| Wirksubstanz | | 4,0 g |
| Carboxymethylcellulose | | 0,1 g |
| p-Hydroxybenzoesäuremethylester | | 0,05 g |
| p-Hydroxybenzoesäurepropylester | | 0,01 g |
| Rohrzucker | | 10,0 g |
| Glycerin | | 5,0 g |
| Sorbitlösung 70 % | | 20,0 g |
| Aroma | | 0,3 g |
| Wasser dest. | ad. | 100,0 ml |

<u>Herstellungsverfahren:</u>

Dest. Wasser wird auf 70°C erhitzt. Hierin wird unter Rühren p-Hydroxybenzoesäuremethylester und -propylester sowie Glycerin und Carboxymethylcellulose gelöst. Es wird auf Raumtemperatur abgekühlt und unter Rühren die Wirksubstanz zugegeben und homogen dispergiert. Nach Zugabe und Lösen des Zuckers, der Sorbitlösung und des Aromas wird die Suspension zur Entlüftung unter Rühren evakuiert.

5 ml Suspension enthalten 200 mg Wirksubstanz.

## Patentansprüche

1.) Als neue Substanz 6-Ethoxy-4-methyl-benzothiazol-2(3H)-on.

2.) Arzneimittel enthaltend 6-Ethoxy-4-methyl-benzothiazol-2(3H)-on neben üblichen Träger- und/oder Hilfsstoffen.

3.) Analgetisch und antipyretisch wirkendes Arzneimittel enthaltend 6-Ethoxy-4-methyl-benzothiazol-2(3H)-on neben üblichen Träger- und/oder Hilfsstoffen.

4.) Verwendung von 6-Ethoxy-4-methyl-benzothiazol-2(3H)-on zur Schmerzbekämpfung und als fiebersenkendes Mittel.

5.) Verfahren zur Herstellung von 6-Ethoxy-4-methyl-benzothiazol-2(3H)-on, dadurch gekennzeichnet, daß

a.) ein Benzothiazol der allgemeinen Formel

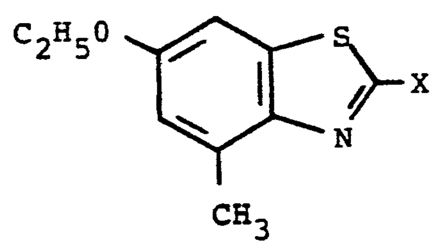

,(II)

in der X ein Chlor- oder Bromatom oder eine bis zu 3 Kohlenstoffatomen enthaltende Alkoxygruupe bedeutet, bei Temperaturen zwischen 0 und 120°C mit Hilfe von wässerigen Mineralsäuren hydrolysiert wird, oder

b.) das 2(3H)-Benzothiazolthion der Formel

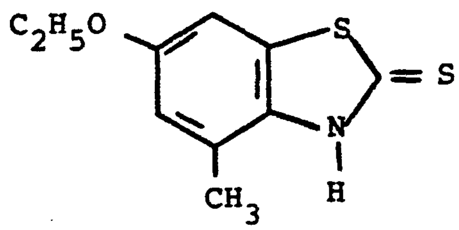

,(III)

bzw. die 2-substituierte Analogverbindung der Formel

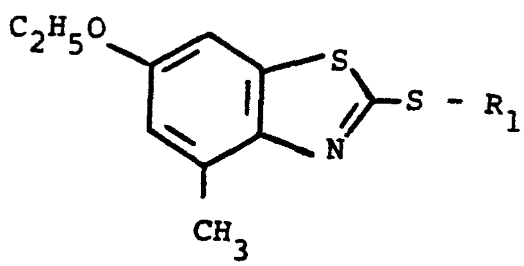

,(IIIb)

in der $R_1$ einen Alkylrest mit 1 bis 3 Kohlenstoffatomen oder einen Benzylrest bedeutet, in wässerig alkalischem Medium oder in Eisessig bei -10 bis +30°C oxidiert und anschließend mittels Säuren bei Temperaturen zwischen 40 und 100°C hydrolisiert wird, oder

c.) der ortho-Mercapto-phenylharnstoff der Formel

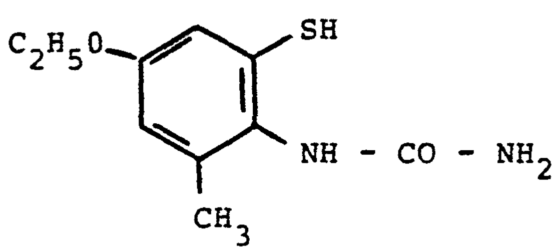

,(IVa)

in Gegenwart einer Säure bei Temperaturen zwischen 40 und 100°C cyclisiert wird, oder

d.) das 2-Aminothiophenol der Formel

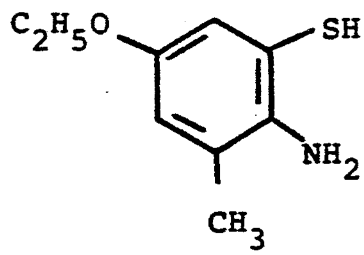

,(V)

mit einem Kohlensäurederivat der allgemeinen Formel

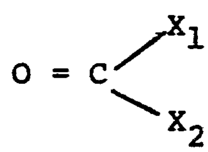

(VI)

in der $X_1$ und $X_2$ gleich oder verschieden sind und Halogenatome oder die 1-Imidazolyl-, 1,2,4-Triazol-4-yl-, 1,2,4-Triazol-1-yl-, Alkoxy mit 1 bis 10 Kohlenstoffatomen, Phenoxy-, p-Tolyloxy-, Benzyloxy- oder die Phenethyloxygruppe oder aber zusammen ein Schwefelatom bedeuten, bei Temperaturen zwischen 0 und 150°C cyclisiert wird, oder

e.) eine Verbindung der allgemeinen Formel

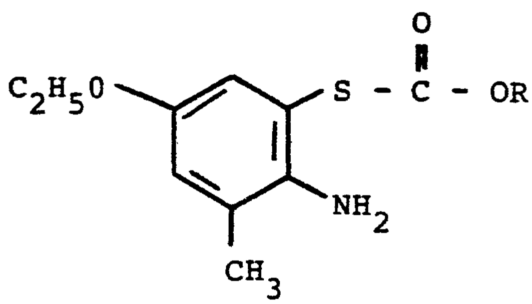

,(VII)

in der R einen Alkylrest mit 1 bis 10 Kohlenstoffatomen, einen Aryl- oder Aralkylrest mit 1 bis 3 Kohlenstoffatomen in der Alkylengruppe bedeutet, in Gegenwart saurer Katalysatoren zwischen 20 und 150°C cyclisiert wird.

6.) Verfahren gemäß Anspruch 5a, dadurch gekennzeichnet, daß die Reaktion in Gegenwart inerter, mit Wasser mischbarer Lösungsmittel bei Temperaturen bis zum Siedepunkt des Reaktionsgemisches durchgeführt wird.

7.) Verfahren gemäß Anspruch 5b, dadurch gekennzeichnet, daß die Oxidation der Verbindung III mit höchstens bis zu 3 Moläquivalenten Chlor oder Brom, mit mindestens 3 Moläquivalenten Wasserstoffperoxid oder mit mindestens 2 Moläquivalenten an Alkalipermanganaten, bezogen auf 1 Mol der Verbindung der Formel III, in Gegenwart von Natriumhydroxid oder Bariumhydroxid als Basen durchgeführt wird und das bei der Oxidation mit Alkalipermanganaten entstehende Zwischenprodukt der Formel

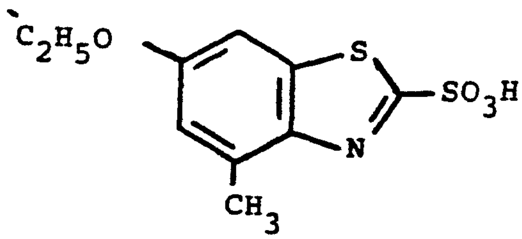

,(IIIa)

anschließend durch Erwärmen auf Temperaturen zwischen 40 und 100°C in Gegenwart wäßriger Mineralsäuren zu dem 6-Ethoxy-4-methyl-benzothiazol-2(3H)-on hydrolysiert wird.

8.) Verfahren gemäß Anspruch 5b, dadurch gekennzeichnet, daß eine Verbindung der allgemeinen Formel IIIb zuerst zu einem Sulfon der allgemeinen Formel

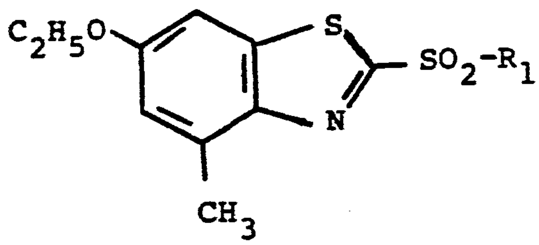

,(IIIc)

in der $R_1$ wie im Anspruch 5b definiert ist, mit Kaliumpermanganat in alkalischer Lösung oder in Eisessig oxidiert und das erhaltene Sulfon anschließend durch Erwärmen auf 40 bis 100°C, gegebenenfalls in Gegenwart von mit Wasser mischbaren Cosolventien, mit wässrigen Mineralsäuren hydrolysiert wird.

9.) Verfahren gemäß Anspruch 5c, dadurch gekennzeichnet, daß die Cyclisierung in Gegenwart wässeriger Mineralsäuren, gegebenenfalls in Gegenwart von Lösungs- oder Verdünnungsmittel, bei Temperaturen zwischen 70 und 100°C erfolgt.

10.) Verfahren gemäß Anspruch 5d, dadurch gekennzeichnet, daß die Umsetzung der Verbindung der Formel V mit einem Kohlensäurederivat der allgemeinen Formel VI in einem Lösungsmittel, gegebenenfalls in Gegenwart zusätzlicher anorganischer oder organischer Basen, bei Temperaturen zwischen 20°C und dem Siedepunkt des Reaktionsgemisches erfolgt.

Europäisches Patentamt

**EUROPÄISCHER TEILRECHERCHENBERICHT,** der nach Regel 45 des Europäischen Patentübereinkommens für das weitere Verfahren als europäiscner Recherchenbericht gilt

0152574

Nummer der Anmeldung

EP 84 11 4982.6

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| A | EP - A1 - 0 039 818 (K. THOMAE GMBH)<br>* Ansprüche 1, 2, 5-8 * | 1-3, 5,7 | C 07 D 277/68<br>A 61 K 31/425 |
| D,A | & DE - A1 - 3 017 977<br>-- | | |
| A | EP - A1 - 0 022 213 (K. THOMAE GMBH)<br>* Ansprüche 1-3, 6, 7, 10 * | 2,3 | |
| D,A | & DE - A1 - 2 927 352<br>-- | | |
| A | EP - A1 - 0 039 483 (HOECHST AG)<br>* Anspruch; Seite 13, Beispiele 17,18 *<br>---- | 5 | |
| | | | RECHERCHIERTE SACHGEBIETE (Int. Cl. 3) |
| | | | C 07 D 277/00 |

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-3, 5-10

Unvollständig recherchierte Patentansprüche: --

Nicht recherchierte Patentansprüche: 4

Grund für die Beschränkung der Recherche: (Verfahren zur chirurgischen oder therapeutischen Behandlung des menschlichen oder tierischen Körpers; (siehe Art. 52 (4) des Europäischen Patentübereinkommens)

| Recherchenort | Abschlußdatum der Recherche | Prüfer |
|---|---|---|
| Berlin | 14-03-1985 | HASS |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1505.1 03 82